# EUROPÄISCHE PATENTANMELDUNG

(11) **EP 2 320 210 A2**
(43) Veröffentlichungstag der Anmeldung: **11.05.2011**
(21) Anmeldenummer: 10185358.8
(22) Anmeldetag: 01.10.2010
(51) Int. Cl.: G01N 17/02, G01N 33/28

(54) **Schutzvorrichtung für ein Kraftfahrzeug**

(30) Priorität: 09.11.2009 DE 102009046521
(71) Anmelder: Robert Bosch GmbH, 70442 Stuttgart (DE)
(72) Erfinder: Repphun, Gernot, 72336 Balingen (DE)

(57) **Zusammenfassung**

Schutzvorrichtung (10) für ein Kraftfahrzeug, mit einem Sensor (13) zur Erfassung einer alterungsabhängigen Eigenschaft eines Kraftstoffes (1), insbesondere eines Dieselkraftstoffes, einer Dosiereinrichtung (20) zum Beimischen eines die Alterung beeinflussenden Additivs (2) und einer Logik zur Ansteuerung der Dosiereinrichtung (20) in Abhängigkeit eines von dem Sensor (13) erfassten Messwertes. Es ist vorgesehen, dass der Sensor ein Sensor (13) zur Erfassung der Korrosion des Kraftstoffes (1) ist.

## Beschreibung

### Stand der Technik

Die Erfindung betrifft eine Schutzvorrichtung für ein Kraftfahrzeug nach dem Oberbegriff des Anspruchs 1.

Eine derartige Schutzvorrichtung ist aus der nachveröffentlichten DE 10 2008 002 356 A1 der Anmelderin bekannt. Die bekannte Schutzvorrichtung weist einen Sensor auf, der eine alterungsabhängige bzw. alterungsbedingte Eigenschaft eines Kraftstoffes erfasst. Sollte hierbei ein bestimmter Grenzwert überschritten werden, so wird eine Dosiereinrichtung angesteuert, welche durch Abgabe eines Additivs die alterungsbedingte Eigenschaft des Kraftstoffes in gewünschter Weise verbessert bzw. abschwächt.

Ferner ist es aus der DE 10 2004 001 422 A1 bekannt, einen Sensor zum Bestimmen der Eigenschaften von Schmier- und/oder Hydraulikölen oder von Bremsflüssigkeiten in einem Kraftfahrzeug einzusetzen. Hierbei kann der Sensor mit dem Bordcomputer signalleitend verbunden werden, um von dem Sensor erhaltene Informationen zu verarbeiten.

Insbesondere moderne Dieselhochdruckeinspritzsysteme sind bzgl. der Korrosionsneigung von alternden Dieselkraftstoffen minderer Qualität insofern kritisch, als sich dadurch zum Beispiel die Abgasgrenzwerte nicht oder nur schwer einhalten lassen bzw. die Bauteile des Dieselhochdruckeinspritzsystems zusätzliche, teilweise aufwändige Maßnahmen erfordern, um mit zur Korrosion neigendem Kraftstoff betrieben werden zu können.

### Offenbarung der Erfindung

Ausgehend von dem dargestellten Stand der Technik liegt der Erfindung die Aufgabe zugrunde, eine Schutzvorrichtung für ein Kraftfahrzeug nach dem Oberbegriff des Anspruchs 1 derart weiterzubilden, dass eine Korrosionsneigung des Kraftstoffes aufgrund der alterungsabhängigen Veränderung der Bestandteile des Kraftstoffes besonders sicher erkannt wird. Diese Aufgabe wird bei einer Schutzvorrichtung für ein Kraftfahrzeug mit den Merkmalen des Anspruchs 1 gelöst. Der Erfindung liegt hierbei die Idee zugrunde, einen Sensor vorzusehen, der die Korrosion des Kraftstoffes erfasst.

Vorteilhafte Weiterbildungen der erfindungsgemäßen Schutzvorrichtung für ein Kraftfahrzeug sind in den Unteransprüchen angegeben. In den Rahmen der Erfindung fallen sämtliche Kombinationen aus zumindest zwei von in den Ansprüchen, der Beschreibung und/oder den Figuren offenbarten Merkmalen.

In einer bevorzugten Ausführungsform der Erfindung ist es vorgesehen, dass eine Dosiereinrichtung zum Beimischen eines die Alterung beeinflussenden Additivs und eine Logik zur Ansteuerung der Dosiereinrichtung vorgesehen sind, die in Abhängigkeit eines von dem Sensor erfassten Messwertes die Dosiereinrichtung ansteuert, wobei die Dosiereinrichtung mit einem Tank zur Speicherung des Additivs verbunden ist. Hierdurch wird eine automatische bzw. selbständige Dosierung eines Additivs bewirkt, ohne dass hierzu der Fahrer eingreifen muss. Ferner wird durch den Tank die Möglichkeit einer Vorratsspeicherung für das Additiv ermöglicht, sodass die Vorrichtung über einen längeren Zeitraum, z.B. über mehrere Jahre, im Einsatz bleiben kann, ohne dass hierzu ständige Kontrollen hinsichtlich des Vorhandenseins des Additivs oder ähnliches erforderlich sind.

Zur Erfassung der Korrosion ist es bevorzugt vorgesehen, dass der Sensor eine elektrochemische Messeinheit aufweist. Mittels einer derartigen elektrochemischen Messeinheit lässt sich die Korrosion des Kraftstoffes relativ einfach und zuverlässig erfassen.

Hierbei ist es in einer konstruktiven Ausgestaltung der elektrochemischen Messeinheit vorgesehen, dass die Messeinheit zumindest zwei Elektroden aufweist, zwischen denen sich der Kraftstoff befindet und, dass die Elektroden im Kraftstofftank des Kraftfahrzeugs angeordnet sind. Durch diese Ausgestaltung wird ein kompakter Aufbau der Vorrichtung ermöglicht, welche somit außerhalb des Kraftfahrzeugtanks keinerlei bzw. nur einen geringen zusätzlichen Bauraum beansprucht.

Zur Verbesserung bzw. Verstärkung der Messsignale des Sensors ist es darüber hinaus in vorteilhafter Weise vorgesehen, dass der Kraftstoff an den Elektroden vorbeiströmt. Dadurch wird nicht nur die Höhe des Messsignals in gewünschter Weise positiv beeinflusst, sondern es wird auch gewährleistet, dass nicht stets derselbe Kraftstoff bzw. dieselbe Teilmenge an Kraftstoff auf Korrosion überprüft wird, sondern das gesamte Kraftstoffvolumen im Tank.

Zur Verbesserung der Messeigenschaften der Elektroden ist es weiterhin vorgesehen, dass die Elektroden plattenförmig ausgebildet sind. Dadurch wird eine Vergrößerung der wirksamen Oberflächen an den Elektroden geschaffen, welches sich positiv auf die Messsignale auswirkt.

Weiterhin kann es in vorteilhafter Weise vorgesehen sein, dass die Dosiereinrichtung über eine Steuereinrichtung zusätzlich Informationen von anderen Sensoren zumindest mittelbar zur Ansteuerung der Dosiereinrichtung als Eingangsgröße erhält. Derartige Informationen können beispielsweise die Häufigkeit bzw. das Datum des letzten Befüllens des Kraftstofftanks betreffen, oder andersartig für die Bewertung der Korrosion des Kraftstoffs relevante Informationen, oder auch sonstige Umweltinformationen, wie beispielsweise die Außentemperatur oder ähnliches.

Eine Tankeinbaueinheit mit einer erfindungsgemäßen Schutzvorrichtung hat den Vorteil, dass sie als Baueinheit komplett zum Beispiel bei einem Tankhersteller gefertigt und montiert werden kann, und dort als Baueinheit vorab geprüft werden kann.

Weitere Vorteile der erfindungsgemäßen Schutzvorrichtung für ein Kraftfahrzeug ergeben sich aus der nachfolgenden Beschreibung bevorzugter Ausführungsbeispiele sowie anhand der Zeichnungen.

Diese zeigen in:
- Fig. 1: eine vereinfachte, schematische Darstellung einer erfindungsgemäßen Schutzvorrichtung für ein Kraftfahrzeug,
- Fig. 2: eine Darstellung zur Verdeutlichung des Messverfahrens einer elektro- chemischen Messeinheit unter Verwendung der Messung der Impedanz zur Feststellung der Korrosion und
- Fig. 3 und 4: Darstellungen zur Verdeutlichung des Messverfahrens unter Verwen- dung eines Potentiostaten als Bestandteil der Messeinheit für die Korro- sion.

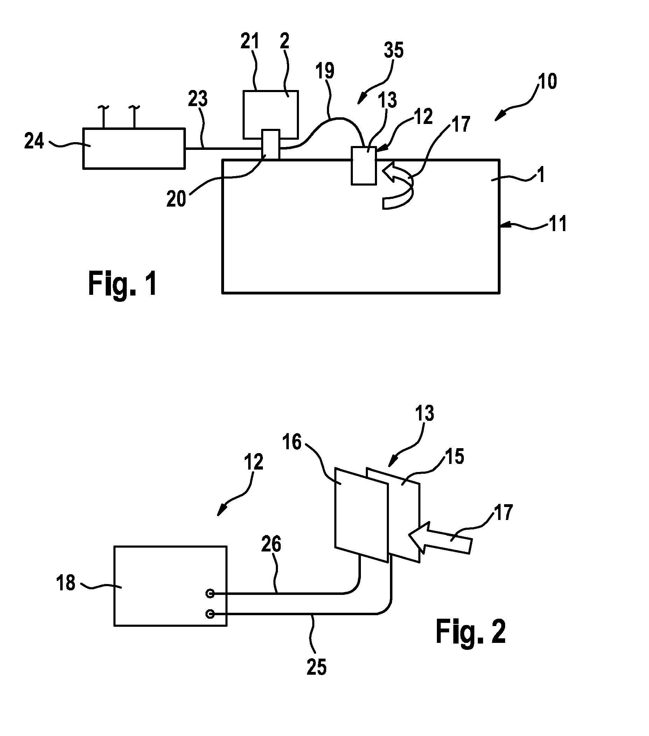

In der Fig. 1 ist eine erfindungsgemäße Schutzvorrichtung 10 für ein im Übrigen nicht dargestelltes Kraftfahrzeug gezeigt. Die Schutzvorrichtung 10 findet hierbei bevorzugt Einsatz in Kraftfahrzeugen mit Dieselhochdruckeinspritzsystemen, z.B. mit so genannten "Common-Rail-Systemen". Besonders relevant ist der Einsatz der erfindungsgemäßen Schutzvorrichtung 10 in Märkten, bei denen die Kraftstoffqualität fraglich ist, da es bei einer mangelnden Kraftstoffqualität unter anderem zu Korrosionsproblemen kommen kann, weil die Korrosionsschutzwirkung des Dieselkraftstoffs nicht gegeben ist, bzw. weil Bestandteile des Dieselkraftstoffs altern, d.h. sich unter Freisetzung von korrosiv wirkenden Säuren zersetzen. Hierbei soll mittels der erfindungsgemäßen Schutzvorrichtung 10 die Korrosionsschutzwirkung des Dieselkraftstoffs im Fahrzeug auf ein hinreichendes Maß gesteigert werden.

Die Schutzvorrichtung 10 ist in Wirkverbindung mit einem Kraftstofftank 11 angeordnet, in dem der Kraftstoff 1 für das Kraftfahrzeug gespeichert ist. Eine elektrochemische Messeinheit 12 weist einen Sensor 13 auf, der innerhalb des Kraftstofftanks 11 angeordnet ist. Wie man insbesondere anhand der Fig. 2 bis 4 erkennt, weist der Sensor 13 zwei in vorzugsweise relativ geringem Abstand zueinander beabstandete, plattenförmige Elektroden 15, 16 auf, die mit einer später noch näher erläuterten Einheit 18 gekoppelt sind, welche Bestand der elektrochemischen Messeinheit 12 ist. Bevorzugt wird zumindest der Zwischenraum zwischen den beiden Elektroden 15, 16 mit Kraftstoff 1 durchströmt, was in den Fig. 1 bis 4 durch die Pfeile 17 dargestellt sein soll.

Die elektrochemische Messeinheit 12 ist über eine erste Leitung 19 mit einer Dosiereinrichtung 20 gekoppelt, welche mit einem Tank 21 zur Speicherung eines Additivs 2 verbunden ist. Bei dem Additiv 2 handelt es sich insbesondere um ein Additiv 2, welches die Korrosion im Kraftstoff 1 herabsetzt, z.B. um Fettsäuremethylester. Hierbei dosiert die Dosiereinrichtung 20 entsprechend einem von der elektrochemischen Messeinheit 12 der Dosiereinrichtung 20 zugeführten Eingangsignal bzw. Messwert mittels einer insbesondere in der Dosiereinrichtung 20 als Ansteuerprogramm ausgebildeten Logik bzw. Algorithmus eine entsprechende Menge des Additivs 2 in den Kraftstoff 1 des Kraftstofftanks 11.

Zusätzlich zur ersten Leitung 19 ist eine zweite Leitung 23 vorgesehen, die mit der Dosiereinrichtung 20 gekoppelt ist und über die der Dosiereinrichtung 20 ebenfalls Eingangssignale zugeführt werden können. Die zweite Leitung 23 ist über eine Steuereinrichtung 24 und Leitungen mit nicht dargestellten Sensoren gekoppelt, welche sonstige relevante Daten sammelt und auswertet, welche bezüglich der Bewertung der Korrosion des Kraftstoffes 1 für die Schutzvorrichtung 10 relevant sind. Hierbei kann es sich z.B. um Daten bezüglich der Umweltbedingungen (z.B. Außentemperaturen) oder aber um fahrerspezifische oder fahrzeugspezifische Daten (z.B. Häufigkeit und Datum der letzten Tankbefüllungen) handeln. All diese Daten können der Dosiereinrichtung 20 über die zweite Leitung 23 zugeführt werden und werden in der Dosiereinrichtung 20 über einen dort vorhandenen Algorithmus bezüglich der Dosiermenge oder des Zeitpunkts der Dosierung des Additivs 2 in den Kraftstofftank 11 berücksichtigt.

In der Fig. 2 ist eine erste Ausführungsform der elektrochemischen Messeinheit 12 dargestellt. Man erkennt die als Arbeitselektrode wirkende Elektrode 15 sowie die als Gegenelektrode wirkende Elektrode 16, welche jeweils über Leitungen 25, 26 mit der Einheit 18 verbunden sind. Hierbei wirkt die Einheit 18 gleichzeitig als Wechselspannungsquelle und als Einrichtung zur Messung der Impedanz. Von der Einheit 18 wird hierbei eine Wechselspannung im Bereich weniger Millivolt an die beiden Elektroden 15, 16 angelegt und die Impedanz gemessen. Aus dem kapazitiven und dem ohmschen Anteil der Impedanz wird bei einer oder mehreren Frequenzen auf die Korrosivität des Kraftstoffes 1 geschlossen und ein entsprechendes Eingangssignal an die Dosiereinrichtung 20 weitergeleitet.

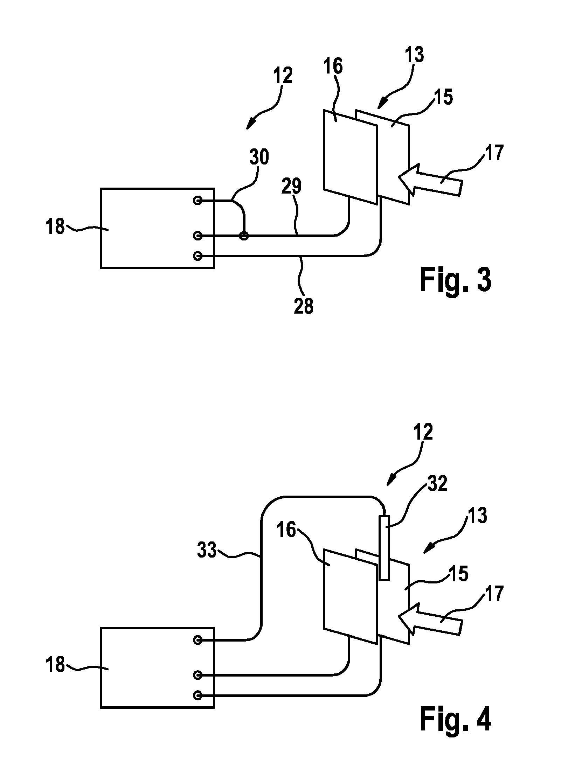

In der Fig. 3 ist eine zweite Ausführungsform der elektrochemischen Messeinheit 12 dargestellt. Hierbei ist die Einheit 18 als Potentiostat ausgebildet, welcher über Leitungen 28, 29 mit den beiden Elektroden 15 und 16 verbunden ist. Hierbei ist wesentlich, dass die eine Elektrode 16 sowohl als Gegenelektrode als auch als sogenannte Referenzelektrode wirkt. Hierzu ist die eine Leitung 29 mit einer Abzweigleitung 30 versehen, welche in dem Fall mit der Einheit 18 zusammenwirkt, wenn die Elektrode 16 als Referenzelektrode dient.

Bei dem in der Fig. 4 dargestellten Ausführungsbeispiel der elektrochemischen Messeinheit 12 ist eine separate, stabförmige Referenzelektrode 32 vorgesehen, welche zwischen den beiden Elektroden 15 und 16 angeordnet ist und über eine Leitung 33 mit der Einheit 18 verbunden ist. Hierbei besteht die Referenzelektrode 32 aus der Elektrode und einem die Elektrode umgebenden Medium, welches mit der Elektrode eine Reaktion eingeht, z.B. eine Silberelektrode in einer Salzsäurelösung. Durch die elektrochemische Reaktion des Silbers mit der Salzsäure entsteht in Abhängigkeit von der Salzsäurekonzentration ein konstantes elektrochemisches Potential. Gegenüber diesem Referenzpotential kann die als Arbeitselektrode wirkende Elektrode 15 durch Anlegen einer Spannung auf ein elektrochemisches Potential gezwungen werden, auf dem eine Korrosionsreaktion stattfindet. Durch die als Potentiostat wirkende Einheit 18 kann der dann entstehende Korrosionsstrom über die als Gegenelektrode wirkende Elektrode 16 anstelle der Referenzelektrode abgeleitet und gemessen werden. Der Korrosionsstrom kann als Maß für die Korrosivität des Kraftstoffes 1 herangezogen werden.

Ergänzend wird erwähnt, dass die Schutzvorrichtung 10 zusammen mit dem Kraftstofftank 11 Bestandteil einer sogenannten Tankeinbaueinheit 35 sein kann, welche als Ganzes vorab bei einem Tankhersteller montiert und als Einheit in das Kraftfahrzeug bei Kraftfahrzeughersteller eingesetzt werden kann.

## Patentansprüche

1. Schutzvorrichtung (10) zur Beeinflussung einer alterungsabhängigen Eigenschaft eines Kraftstoffes (1), insbesondere eines Dieselkraftstoffes, in einem Kraftfahrzeug, mit einem Sensor (13) zur Erfassung der alterungsabhängigen Eigenschaft des Kraftstoffes (1),
**dadurch gekennzeichnet,**
**dass** der Sensor ein Sensor (13) zur Erfassung der Korrosion des Kraftstoffes (1) ist.

2. Schutzvorrichtung nach Anspruch 1,
**dadurch gekennzeichnet,**
**dass** Mittel zur Beeinflussung der alterungsabhängigen Eigenschaft des Kraftstoffes (1) vorgesehen sind, wobei die Mittel eine Dosiereinrichtung (20) zum Beimischen eines die Alterung beeinflussenden Additivs (2) und eine Logik zur Ansteuerung der Dosiereinrichtung (20) umfassen, die in Abhängigkeit eines von dem Sensor (13) erfassten Messwertes die Dosiereinrichtung (20) ansteuert, wobei die Dosiereinrichtung (20) mit einem Tank (21) zur Speicherung des Additivs (2) verbunden ist.

3. Schutzvorrichtung nach Anspruch 1 oder 2,
**dadurch gekennzeichnet,**
**dass** der Sensor (12) eine elektrochemische Messeinheit (13) aufweist.

4. Schutzvorrichtung nach Anspruch 3,
**dadurch gekennzeichnet,**
**dass** die Messeinheit (13) zumindest zwei Elektroden (15, 16, 32) aufweist, zwischen denen sich Kraftstoff (1) befindet und, dass die Elektroden (15, 16, 32) in einem Kraftstofftank (11) angeordnet sind.

5. Schutzvorrichtung nach Anspruch 4,
**dadurch gekennzeichnet,**
**dass** der Kraftstoff (1) an den Elektroden (15, 16, 32) vorbeiströmt.

6. Schutzvorrichtung nach Anspruch 4 oder 5,
**dadurch gekennzeichnet,**
**dass** zwei der Elektroden (15, 16) mit einer als Wechselspannungsquelle wirkenden Einheit (18) verbunden sind und, dass die Impedanz bei wenigstens einer Frequenz der Wechselspannung als Messwert für das Maß der Korrosion des Kraftstoffes (1) herangezogen wird.

7. Schutzvorrichtung nach Anspruch 4 oder 5,
**dadurch gekennzeichnet,**
**dass** zwei der Elektroden (15, 16) mit einer als Gleichspannungsquelle wirkenden Einheit (18) verbunden sind und, dass die Gleichspannungsquelle als Potentiostat ausgebildet ist.

8. Schutzvorrichtung nach Anspruch 7,
**dadurch gekennzeichnet,**
**dass** eine zusätzliche Referenzelektrode (32) vorhanden ist, die mit dem Potentiostaten verbunden ist.

9. Schutzvorrichtung nach einem der Ansprüche 4 bis 8,
**dadurch gekennzeichnet,**
**dass** zwei der Elektroden (15, 16) plattenförmig ausgebildet sind.

10. Schutzvorrichtung nach einem der Ansprüche 2 bis 9,
**dadurch gekennzeichnet,**
**dass** die Dosiereinrichtung (20) über eine Steuereinrichtung (24) zusätzlich Informationen von anderen Sensoren zumindest mittelbar zur Ansteuerung der Dosiereinrichtung (20) als Eingangsgröße erhält.

11. Tankeinbaueinheit (35), umfassend eine Schutzvorrichtung (10) nach einem der Ansprüche 1 bis 10 und einen Tank (21) zur Speicherung eines die Alterung des Kraftstoffes (1) beeinflussenden Additivs (2).
